# EUROPÄISCHE PATENTSCHRIFT

(11) **EP 3 697 349 B1**
(45) Veröffentlichungstag und Bekanntmachung des Hinweises auf die Patenterteilung: **31.12.2025**
(21) Anmeldenummer: 18783480.9
(22) Anmeldetag: 09.10.2018
(51) Int. Cl.: A61F 2/64

(54) **ORTHOPÄDIETECHNISCHE EINRICHTUNG UND DÄMPFER**
ORTHOPAEDIC DEVICE AND DAMPER
DISPOSITIF TECHNIQUE ORTHOPÉDIQUE ET AMORTISSEUR

(30) Priorität: 18.10.2017 DE 102017124298
(43) Veröffentlichungstag der Anmeldung: 26.08.2020
(73) Patentinhaber: Ottobock SE & Co. KGaA, 37115 Duderstadt (DE)
(72) Erfinder: KUNZE, René, 37077 Göttingen (DE)
(74) Vertreter: Gramm, Lins & Partner Patent- und Rechtsanwälte PartGmbB
(86) Internationale Anmeldenummer: PCT/EP2018/077439
(87) Internationale Veröffentlichungsnummer: WO 2019/076680

(56) Entgegenhaltungen:
- CN-U- 201 768 054
- DE-A1- 102013 016 800
- DE-B3- 102014 006 688
- US-A1- 2013 268 092

## Beschreibung

Die Erfindung betrifft eine orthopädietechnische Einrichtung mit einem Kolben zur Anordnung in einem Dämpfer mit einem Zylinder, in dem der Kolben relativ zu dem Zylinder verlagerbar gelagert ist, wobei der Kolben einen Grundkörper aufweist, an dessen Außenseite zumindest ein Kontaktelement angeordnet ist, das in Richtung auf den Zylinder verlagerbar und mit einem elastischen Element gekoppelt ist. Die Erfindung betrifft ebenfalls einen Dämpfer mit einem Zylinder und einem solchen Kolben.

Dämpfereinrichtungen, insbesondere in prothetischen und orthetischen Systemen oder Einrichtungen, können als hydraulische oder pneumatische Dämpfer ausgebildet sein, bei denen ein Kolben innerhalb eines Zylinders über eine Kolbenstange mit einer Kraft beaufschlagt wird. Das hydraulische oder pneumatische Medium wird durch den Kolben aus einer Kolbenkammer verdrängt, beispielsweise durch ein Ventil oder eine Drossel umgelenkt und in einen Ausgleichsbehälter oder aber in ein Volumen, das hinter dem Kolben liegt, umgeleitet. Durch die Durchleitung des Mediums durch eine Drossel wird kinetische Energie dissipiert und Bewegungsenergie aus dem System genommen.

Pneumatische und hydraulische Systeme benötigen einen relativ hohen Aufwand, um gegenüber Leckagen abgesichert zu sein. Bewegungsdichtungen sind vorhanden, ebenso ist es notwendig, Drosseln, Ventile, Rückschlagelemente und dergleichen vorzusehen, auszulegen und zu montieren, so dass diese Dämpfereinrichtungen relativ aufwendig sind.

Sowohl in der Prothetik als auch in der Orthetik besteht jedoch auch ein Bedarf an einfachen Dämpfern und zugehörigen Bauteilen, mit denen es möglich ist, auf preiswerte und robuste Art und Weise eine einfache Dämpfung, beispielsweise eine Schwungphasendämpfung in einem Kniegelenk, zu verwirklichen.

Aus der CN 201768054 U ist ein Prothesenkniegelenk mit einem Feststoffdämpfer bekannt, bei dem eine Spreizhülse in einem Zylinder geführt ist. Über eine Gewindestange kann der Abstand zwischen zwei Konen verändert und die Spreizhülse aufgeweitet werden.

Die GB 2 499 859 A betrifft eine Prothese für untere Extremitäten mit einem Befestigungsabschnitt, einem Unterschenkelabschnitt, einem Fußabschnitt, einem Kniegelenk, das den Befestigungsabschnitt und den Unterschenkelabschnitt schwenkbar miteinander verbindet, und einem Knöchelgelenksabschnitt, das den Unterschenkelabschnitt und den Fußabschnitt schwenkbar miteinander verbindet. Eine Dämpfereinrichtung ist zur Dämpfung der Schwenkbewegung dem jeweiligen Gelenk zugeordnet. Die Dämpfereinrichtung ist sensorgesteuert und kann einen hydraulischen und einem pneumatischen Abschnitt aufweisen. Zur Veränderung der Dämpfereigenschaften werden Ventile geöffnet oder geschlossen.

Die US 2013/0264157 A1 betrifft einen pneumatischen Dämpfer, der einstellungsfrei arbeitet. In einem Zylinder ist ein Kolben angeordnet, der ein Rückschlagventil aufweist, ein gleichgerichtetes Rückschlagventil ist in dem Zylindergehäuse angeordnet. Vergrößert sich das Volumen innerhalb der Kammer, die dem zylinderseitigen Rückschlagventil zugeordnet ist, öffnet dieses, verringert sich das Volumen, öffnet sich das Rückschlagventil in dem Kolben.

Die US 2010/0049332 A1 betrifft ein Prothesenbein mit einem Zylinder, in dem ein Kolben längsbeweglich an einer Kolbenstange gelagert ist. Die Kolbenstange ist an einem Außenrohr gelagert, der untere Teil des Zylinders ist an einem Innenrohr befestigt. Das Innenrohr ist in dem Außenrohr eingeführt. Innerhalb des Zylinders ist ein pneumatisches oder hydraulisches Medium angeordnet, das Innenrohr und das Außenrohr bilden eine Aufnahme für ein Torsionselement aus. Das Außenrohr kann um das Innenrohr herum gedreht werden. Das Torsionselement weist ein Elastomer auf, das an dem Außenrohr befestigt ist.

Aus der DE 10 2014 006 688 A1 sind ein Kolben zur Anordnung in einem Dämpfer und ein Dämpfer für eine orthopädietechnische Einrichtung mit einem Zylinder bekannt, in dem der Kolben relativ zu dem Zylinder verlagerbar gelagert ist und einen Grundkörper aufweist. An oder in dem Grundkörper ist zumindest ein Verformungselement aus einem bevorzugt elastischen, inkompressiblem Material angeordnet, dem zumindest eine verlagerbare Druckeinrichtung zugeordnet ist, die zumindest partiell auf das zumindest eine Verformungselement einwirkt, wobei sich das Verformungselement beim Aufbringen einer Druckkraft an einen von der Krafteinleitungsstelle verschiedenen Ort zumindest teilweise nach außen wölbt.

Die DE 10 2013 016 800 A1 betrifft eine Dämpfereinrichtung mit einem Kolben, in dem ein bewegliches Verschlusselement gelagert ist, um eine Pneumatikströmung zu erlauben oder zu unterbinden, um so eine positionsunabhängige und richtungsgesteuerte Entlüftung zu bewirken. Ein Verschlusselement liegt an einer Zylinderwand an, um einen Strömungskanal zu öffnen oder zu schließen. Ein Stellelement kann ein Spannelement gegen die Zylinderwand drücken. In einem Ausführungsbeispiel ist gezeigt, dass das Spannelement über das Stellelement gegen das Kontaktelement drückt und damit die Vorspannung des Kolbenringes einstellt.

Aufgabe der vorliegenden Erfindung ist es, eine orthopädietechnische Einrichtung mit einem Kolben für eine Dämpfereinrichtung und einen Dämpfer als solchen bereitzustellen, mit denen auf einfache Weise eine Einstellung der Widerstände erreicht und der eingestellte Widerstand über einen langen Zeitraum aufrechterhalten werden kann.

Erfindungsgemäß wird diese Aufgabe durch einen Dämpfer mit den Merkmalen des Hauptanspruches und eine orthopädietechnische Einrichtung mit den Merkmalen des nebengeordneten Anspruchs erreicht. Vorteilhafte Ausgestaltungen und Weiterbildungen der Erfindung sind in den Unteransprüchen, der Beschreibung sowie den Figuren offenbart.

Der erfindungsgemäße Dämpfer mit einem Kolben und einem Zylinder, in dem der Kolben relativ zu dem Zylinder verlagerbar gelagert ist, wobei der Kolben einen Grundkörper aufweist, an dessen Außenseite zumindest ein Kontaktelement angeordnet ist, das in Richtung auf den Zylinder verlagerbar und mit einem elastischen Element gekoppelt ist, sieht vor, dass das elastische Element über eine Einstellvorrichtung in Verlagerungsrichtung des Kontaktelementes vorspannbar ist.

Durch die Vorspannung des elastischen Elements kann eine konstante oder nahezu konstante Kraft auf das Kontaktelement ausgeübt werden, so dass das Kontaktelement mit einer konstanten Kraft auf die Zylinderinnenwand gedrückt wird und dadurch Toleranzen und Materialverlust durch Abrieb ausgleicht. Weiterhin wird trotz Abrieb eine konstante Reibung sowohl zwischen dem Kontaktelement und dem Zylinder als auch zwischen der gegenüberliegenden Fläche des Grundkörpers mit der Zylinderinnenwand ausgeübt. Die dem Kontaktelement gegenüberliegende Fläche stellt das Widerlager oder die Gegenkraft bereit.

An der gegenüberliegenden Fläche zur Kontaktfläche am Grundkörper kann ein zusätzliches Reibelement angebracht sein, welches mit dem Grundkörper verbunden ist, insbesondere austauschbar daran festgelegt ist. Das Reibelement kann etwas abgeflacht gegenüber dem Radius des Grundkörpers ausgebildet sein, um eine größere Anlagefläche an der Zylinderinnenwand zu erzeugen.

Das Kontaktelement, bzw. die Kontaktfläche ist bevorzugt ausschließlich quer zu der Bewegungsrichtung des Kolbens verfahrbar gelagert und fährt in Richtung auf die Zylinderwand aus dem Grundkörper heraus oder von der Zylinderwand weg in den Grundkörper hinein. Das Kontaktelement kann auch schräg aus dem Kolben fahren, sofern die Kontaktfläche parallel zu der Zylinderwand bleibt. Somit kann die Einstellvorrichtung nicht nur quer zur Bewegungsachse angeordnet sein, sondern auch schräg dazu. Die Einstellvorrichtung kann als ein Exzenter ausgebildet sein.

In der Erfindung ist vorgesehen, dass die Einstellvorrichtung als eine in und entgegen der Verlagerungsrichtung des Kontaktelementes verlagerbare Einstellschraube ausgebildet ist, so dass das elastische Element in Verlagerungsrichtung des Kontaktelementes vorspannbar ist. Über insbesondere die Einstellschraube, aber auch über andere Einstellvorrichtungen, kann das elastische Element in und entgegen der Verlagerungsrichtung des Kontaktelementes veränderlich vorgespannt werden, da die Einstellvorrichtung eine Verlagerung in Verlagerungsrichtung bzw. entgegen der Verlagerungsrichtung des kontaktierten Elementes ausführt oder bewirkt und damit das elastische Element spannt oder entspannt. Über die Einstellvorrichtung wird somit das elastische Element so eingestellt, dass es mit der gewünschten Kraft mittelbar oder unmittelbar entgegen das Kontaktelement drückt und dieses auch in Richtung auf die Zylinderwandung vorspannt. Über die Einstellvorrichtung oder Einstellschraube ist es somit möglich, das elastische Element wie gewünscht zu spannen, so dass die über das elastische Element aufgebrachte, einstellbare Kraft auf das Kontaktelement übertragen wird. Über die dann zwischen dem Kontaktelement und der Zylinderwandung auftretende Reibkraft ist es möglich, eine unterschiedliche Dämpfung zu bewirken. Um eine Kraft mit dem Kontaktelement auf den Zylinder auszuüben, wird der Grundkörper auf der dem Kontaktelement gegenüberliegenden Seite, also mit einer Gegenfläche gegen den Zylinder gedrückt. Der Dämpfer aus Kolben und Zylinder dient beispielsweise zur Steuerung einer Bewegungsphase, insbesondere zur Steuerung der Schwungphase in einem künstlichen Kniegelenk, beispielsweise in einer Orthese oder Prothese. Je nach Vorspannung des elastischen Elementes, die durch die jeweilige Position der Einstellvorrichtung oder Einstellschraube in oder entgegen Verlagerungsrichtung des Kontaktelementes bestimmt wird, werden unterschiedliche Reibkräfte erzeugt. Durch die Zwischenschaltung des elastischen Elementes ist es möglich, dass ein möglicher Verschleiß während des Betriebes des Kolbens in dem Dämpfer durch das elastische Element kompensiert wird, so dass über eine vergleichsweise lange Zeit eine konstante oder nahezu konstante Reibkraft erzeugt werden kann. Durch die Ausgestaltung über eine in einem Gewinde angeordnete Einstellschraube ist es möglich, eine sehr einfache und preiswerte konstruktive Lösung bereitzustellen, mit der eine Feineinstellung der Vorspannkraft über das elastische Element und damit auch der aufgebrachten Reibkraft zwischen dem Kontaktelement und der Zylinderwand und dementsprechend mit der Gegenfläche und der Zylinderwand erfolgen kann.

Das elastische Element kann in einer Weiterbildung der Erfindung als eine Tellerfederanordnung, eine Schraubentellerfeder, eine Schraubenfeder und/oder ein Elastomer ausgebildet sein. Ebenfalls ist es möglich eine Kombination aus Tellerfederanordnung, Schraubenfederanordnung, Schraubenfeder und/oder Elastomerfeder in jeglicher Variation vorzusehen, so dass die jeweils konstruktiv bedingten Vorteile der jeweiligen Ausgestaltung des elastischen Elementes ausgenutzt werden können. Tellerfederanordnungen und Schraubentellerfedern haben den Vorteil, über einen vergleichsweise kurzen Federweg eine hohe Federsteifigkeit bereitzustellen. Ebenfalls wird bei einem Federbruch in der Regel die grundsätzliche Funktionsfähigkeit der Feder nicht oder nur geringfügig beeinträchtigt. Eine Schraubenfeder oder eine Wendelfeder hat den Vorteil, dass sie als Standardbauteil preiswert und leicht verfügbar ist. Eine Ausgestaltung als Elastomer, beispielsweise ein Zylinder aus einem elastischen Material, hat den Vorteil einer vergleichsweise preiswerten Herstellung, jedoch den Nachteil eines vergleichsweise kurzen Federweges. Als weitere Ausgestaltung des elastischen Elementes kann dieses als pneumatisches Element ausgebildet sein, beispielsweise als Druckkissen oder elastischer Schaum, der offenporig oder geschlossenporig ausgebildet sein kann.

In einer Weiterbildung der Erfindung ist es vorgesehen, dass eine Aufnahmeeinrichtung für das elastische Element an dem Kontaktelement angeordnet, befestigt oder ausgebildet ist. Über die Aufnahmeeinrichtung ist es möglich, das elastische Element sicher innerhalb des Kolbens und des Grundkörpers anzuordnen und gegebenenfalls eine Vormontage des elastischen Elementes innerhalb der Aufnahmeeinrichtung auszuführen, um eine erleichterte Kopplung mit dem Kontaktelement vornehmen zu können. Die Aufnahmeeinrichtung kann einstückig an dem Kontaktelement angeformt sein, beispielsweise im Rahmen eines Urformverfahrens wie dem Spritzgießen. Dabei kann zur Einstellung unterschiedlicher Materialpaarungen und zur Ausnutzung unterschiedlicher Materialeigenschaften ein Zweikomponenten-Spritzgießverfahren angewendet werden, um das Kontaktelement mit einer materialtechnisch angepassten Aufnahmeeinrichtung auszugestalten. Alternativ kann die Aufnahmeeinrichtung an dem Kontaktelement befestigt sein, so dass eine zwei oder mehrteilige Ausgestaltung des Kontaktelementes zusammen mit der Aufnahmeeinrichtung vorliegt. Neben einer dauerhaften Befestigung, gegebenenfalls auch einer lösbaren Befestigung des Kontaktelementes an der Aufnahmeeinrichtung, ist es möglich, eine Anordnung des Kontaktelementes an der Aufnahmeeinrichtung vorzusehen, so dass das Kontaktelement an der Aufnahmeeinrichtung anliegt und die Druckkraft überträgt. Über die Aufnahmeeinrichtung können die Druckkräfte, die von dem elastischen Element in Richtung auf das Kontaktelement ausgeübt werden, über eine größere Fläche verteilt werden, so dass eine vergleichmäßigte Anlage des Kontaktelementes an der Innenseite des Zylinders bereitgestellt werden kann.

Die Aufnahmeeinrichtung ist dazu verlagerbar, insbesondere verschieblich in dem Grundkörper angeordnet und kann eine Ausnehmung aufweisen, in der das elastische Element eingeführt ist. Über die Ausnehmung ist es möglich, dass das elastische Element seitlich geführt wird. Die Ausnehmung kann insbesondere einen Tellerfederstapel aufnehmen und die Zuordnung der einzelnen Tellerfederelemente zueinander sicherstellen. Bei einer Ausgestaltung des elastischen Elementes als Schraubentellerfeder, Schraubenfeder oder einem Kunststoffelement dient die Ausnehmung insbesondere als Sicherung gegen ein Wegknicken bei einer Aufbringung einer axial wirkenden Kraft zur Vorspannung des elastischen Elementes in Richtung auf die Zylinderwand.

Das Kontaktelement kann den Grundkörper um mehr als die Hälfte des Grundkörperumfanges umgeben, so dass bei einer Druckkraft nach außen in Richtung auf die Zylinderwand die jeweiligen Enden des Kontaktelementes an der Oberfläche des zylindrischen Grundkörpers entlanggleiten und dadurch eine Auswärtsbewegung oder Aufweitbewegung der Enden oder Endbereiche des Kontaktelementes bewirken, wodurch eine vergrößerte Reibungsfläche des Kontaktelementes mit der Zylinderwand bereitgestellt wird. Hierzu ist es vorgesehen, dass der Grundkörper des Kolbens zumindest im Bereich der Anlage des Kontaktelementes im Wesentlichen kreisförmig ausgebildet ist oder einen kreisförmigen Querschnitt aufweist und das Kontaktelement eine korrespondierende Formgestaltung aufweist. Grundsätzlich besteht die Aufweitfunktion auch bei eckigen oder polygonalen Querschnitten des Kolbens und einer entsprechenden Form des Kontaktelementes. Sofern das Kontaktelement bei einem kreisförmigen Querschnitt des Kolbens sich nicht über die Hälfte des Grundkörperumfanges erstreckt, also kleiner als ein Halbkreis ist, findet keine Aufweitung an den Außenkanten oder den einander gegenüberliegenden Enden des Kontaktelementes statt, und das Kontaktelement wird ohne weitere Deformation gegen die Zylinder gedrückt.

Das Kontaktelement kann sich über mehr als die Hälfte der Höhe des Grundkörpers des Kolbens erstrecken, um eine große Reibfläche bereitzustellen. Dazu kann die Aufnahmeeinrichtung als formstabiles Bauteil ausgebildet sein, auf dem das Kontaktelement an der Außenseite angeordnet ist.

Die Einstellvorrichtung ist bevorzugt in dem Grundkörper gelagert, beispielsweise in einem metallischen Gewindeeinsatz, um eine Einstellung der Vorspannung durch eine Verstellung der Einstellvorrichtung in Gestalt einer Schraube ausführen zu können, ohne dass die Funktionsweise des Dämpfers durch herausstehende Komponenten oder ähnliches beeinträchtigt wird.

Eine Weiterbildung der Erfindung sieht vor, dass eine Reibkraft für die Dämpfung einer Bewegung zwischen dem Kolben und dem Zylinder erzeugt wird. Insbesondere wird die Reibkraft über die Oberfläche des Kontaktelementes, dessen Gegenfläche an dem Grundkörper und der Zylinderinnenwand erzeugt.

Die Erfindung betrifft ebenfalls eine orthopädietechnische Einrichtung mit einem Dämpfer mit einem Zylinder und einem beweglich darin gelagerten Kolben, so wie er oben beschrieben worden ist. Insbesondere ist ein solcher Dämpfer in einer Orthese oder Prothese, insbesondere in einem Orthesenkniegelenk oder Prothesenkniegelenk einsetzbar und eingesetzt, um dort insbesondere die Schwungphase zu steuern.

Die Einstellschraube kann auch auf einen Aufspreizkörper wirken, der mit dem Kontaktelement gekoppelt ist, um in der jeweils gewünschten Richtung Druck auf das Kontaktelement ausüben zu können. Es können auch mehrere Einstellschrauben und Kontaktelemente sowie elastische Elemente in und an dem Kolben angeordnet sein, um eine Feineinstellung, Justierung oder Kalibrierung und Anpassung an die Wünsche z.B. des Prothesen- oder Orthesenträgers sowie eine Vergleichmäßigung der Abstützung des Kolbens in dem Zylinder zu erreichen.

Der Grundkörper besteht vorteilhafterweise aus einem formstabilen Werkstoff, beispielsweise einem hochfesten Kunststoff oder einem Leichtmetall, und umgibt das elastische Element zumindest teilweise, um eine Führung bereitzustellen. Der Kolben kann rotationssymmetrisch ausgebildet sein, wobei das Kontaktelement ebenfalls im Wesentlichen der Kontur des Kolbens folgend ausgebildet ist.

Das elastische Element kann zumindest teilweise aus einem Polymerwerkstoff hergestellt sein, insbesondere aus einem TPU, so dass eine nahezu beliebige Formgebung ausgenutzt werden kann, um die Vorspannkraft durch die Einstellschraube aufzunehmen und weiterzuleiten.

Das Kontaktelement kann als eine aufweitbare Abdeckung des Grundkörpers ausgebildet sein, so dass zunächst vermieden wird, dass ein direkter Reibungskontakt zwischen dem elastischen Element und der Zylinderwandung besteht. Dadurch ist es möglich, dass das Kontaktelement als Verschleißteil ausgebildet sein kann und bei Bedarf ausgewechselt wird. Das Kontaktelement kann auch über eine Scharniereinrichtung an dem Grundkörper verschwenkbar befestigt sein, wobei die Scharniereinrichtung beispielsweise als ein Filmscharnier ausgebildet sein kann. Bei einer Ausgestaltung des Kolbens als Kunststoffteil ist es möglich, den Kolben als Ganzes in einem einzigen Arbeitsgang herzustellen. Dadurch ist es auch möglich, relativ komplexe Formen einfach herzustellen und lediglich die Einstellschraube und das elastische Element als separate Bauteile vorzusehen. Dadurch ist es möglich, Bauteile und Montageschritte einzusparen.

An dem Kolben und/oder dem Kontaktelement kann zumindest eine Abflachung ausgebildet sein, die bevorzugt in dem Bereich der Wirkrichtung des elastischen Elementes positioniert ist. Es können zwei oder mehrere Abflachungen ausgebildet werden, bevorzugt einander gegenüberliegend, um eine gleichmäßige Anlage der Komponenten aneinander und eine gleichmäßige Ausbildung der Anpresskräfte und damit der Reibungskräfte zu erzeugen. Durch die Abflachung oder die Abflachungen, beispielsweise an dem Kontaktelement und der Gegenfläche, können aktive Reibflächen zwischen dem Kolben, dem Kontaktelement und dem Zylinder in Umfangsrichtung neben der Abflachung ausgebildet werden. Die Krümmungsradien der Kontaktflächen des Kontaktelementes und der Gegenfläche des Kolbens können auf den Innendurchmesser des Zylinders verbessert abgestimmt werden. Wenn der Kolben einen geringfügig kleineren Radius als der Zylinder aufweist, würde ohne Abflachung eine erhöhte Anpressung in dem Bereich der Wirkrichtung des elastischen Elementes an dem Kontaktelement und der Gegenfläche auftreten. Zudem würden sich nahezu linienförmige Anlageflächen ausbilden. Durch die Abflachungen werden die Reibkräfte gleichmäßiger um den Umfang verteilt. Bereiche erhöhter Reibkräfte finden sich bevorzugt seitlich, also in Umfangsrichtung neben den Abflachungen, die bevorzugt in dem Bereich der Wirkrichtung des elastischen Elementes angeordnet ist oder sind. Eine Anpassung der Reibflächen des Kolbens an den Zylinder kann auch über elastische Verformungen des Kontaktelementes und ggf. der Gegenfläche erfolgen.

Der erfindungsgemäße Dämpfer mit einem Zylinder und einem beweglich darin gelagerten Kolben, wie er vorstehend beschrieben ist, ermöglicht ein einfaches Einstellen der Dämpfung durch eine Veränderung der Position der Einstellschraube und ist besonders einfach aufgebaut. Aufgrund der Festkörperdämpfung über mechanische Reibung ist es nicht notwendig, Dichtungen für Fluide vorzusehen.

Nachfolgend werden Ausführungsbeispiele der Erfindung anhand der beigefügten Figuren näher erläutert. Es zeigen:
- Figur 1 -: eine perspektivische Außenansicht eines Kolbens;
- Figur 2-: eine Schnittdarstellung eines Kolbens;
- Figur 3 -: eine Schnittdarstellung einer Detailansicht;
- Fig. 4 -: eine Schnittdarstellung eines Kolbens in einem Prothesenkniegelenk;
- Fig. 5 -: eine Gesamtansicht eines Prothesenbeines; sowie
- Fig. 6 -: eine Darstellung eines Kolbens im Horizontalschnitt.

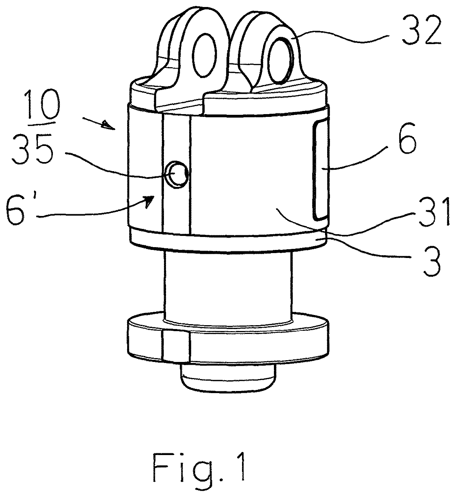

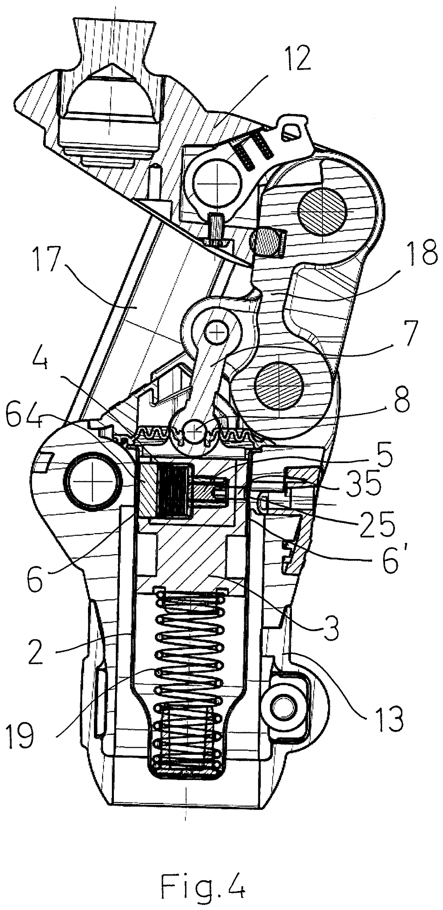

In der Figur 1 ist in einer perspektivischen Darstellung der Außenansicht eines Kolbens 10 mit einem Grundkörper 3 dargestellt, der zumindest eine Außenseite 31 aufweist. An dem Kolben 10 sind an der oberen Endfläche des Kolbens Aufnahmen 32 für einen Kolbenbolzen 8 ausgebildet, über den eine Kolbenstange 7 mit einer weiteren orthopädietechnischen Komponente gekoppelt werden kann. Sowohl der Bolzen 8 als auch die Kolbenstange 7 sind in der Figur 4 gezeigt. Über die Kolbenstange 7 werden Druck- und Zugkräfte von der Komponente auf den Kolben 10 übertragen, so dass dieser längsbeweglich in einem Zylinder 2, der in der Figur 4 dargestellt ist, verlagert werden kann. Der Zylinder 2 ist an einer anderen Komponente der orthopädietechnischen Einrichtung, beispielsweise einer Prothese oder Orthese angeordnet, so dass bei einer Relativbewegung der beiden unterschiedlichen Komponenten der orthopädietechnischen Einrichtung eine Relativverlagerung des Kolbens 10 innerhalb des Zylinders 2 erfolgt. Bei einer Anordnung beispielsweise an einer Prothese oder Orthese der unteren Extremität im Bereich eines Kniegelenkes wird beispielsweise die Kolbenstange 7 an dem Oberteil oder dem Oberschenkel zugeordneten Bauteil und der Zylinder 2 an dem Unterteil oder der dem Unterschenkel zugeordneten Komponente angeordnet, so dass bei einer Extension oder Flexion des künstlichen Kniegelenkes der Kolben 10 in den Zylinder 2 ausfährt oder einfährt.

An der Außenseite 31 des Kolbens 10 ist ein Kontaktelement 6 angeordnet, das sich in seiner Höhe über mehr als die Hälfte der Höhe des Grundkörpers 3 erstreckt. Das Kontaktelement 6 ist an der Außenseite 31 angeordnet und im Wesentlichen korrespondierend zu dem Umfang des Kolbens 10 ausgebildet, weist also eine Form auf, die einem Teil der Mantelfläche eines Kreiszylinders entspricht. In der Außenseite 31 des Grundkörpers 3 ist eine Vertiefung eingearbeitet, die im Wesentlichen der Dicke des Kontaktelementes 6 entspricht, so dass das Kontaktelement 6 bei der Montage im Wesentlichen bündig mit der Außenfläche 31 des Kolbens 10 abschließt. Dies erleichtert oder ermöglicht das Einführen des Kolbens 10 zusammen mit dem Kontaktelement 6 in den Zylinder 2.

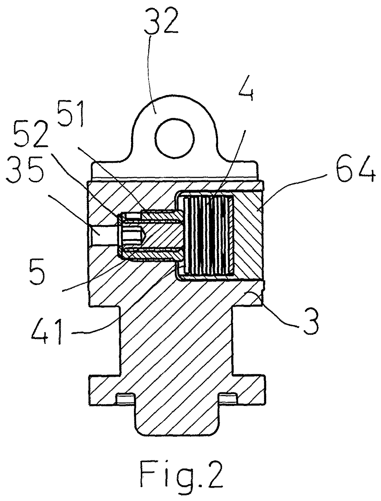

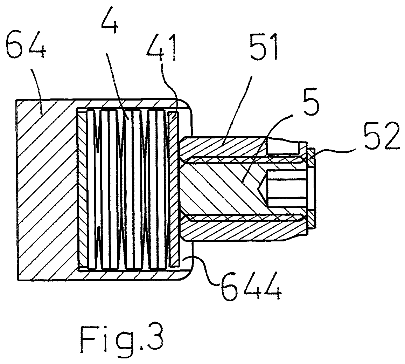

Innerhalb des Kolbens 10 ist in dem Grundkörper 3 eine Bohrung 35 eingearbeitet oder ausgebildet. Die Bohrung 35 erstreckt sich radial nach innen und ermöglicht einen Zugang zu einer Einstellschraube 5, die in der Querschnittsansicht gemäß Figur 2 oder Figur 3 zu erkennen ist. Die Bohrung 35 ist in dem massiven Grundkörper 3 des Kolbens 10 ausgebildet und erweitert sich in Radialrichtung, um dort einen Gewindeeinsatz 51 aufzunehmen, in den die Einstellschraube 5 in Gestalt einer Madenschraube eingeschraubt ist. In der Einstellschraube 5 ist an einem Ende ein Innensechskant ausgebildet, so dass über ein entsprechendes Werkzeug durch eine Rotation der Einstellschraube 5 diese in Richtung auf die Zylinderwand oder von dieser weg, also in beiden radialen Richtungen, verlagert werden kann.

In dem Bereich der Bohrung 35 ist der Grundkörper 3 abgeflacht, um eine Verteilung der Reibkräfte auf die Gegenfläche 6' des Kolbens 10 zu bewirken. An dem Ende der Einstellschraube 5 kann eine Scheibe 52 oder eine Halteeinrichtung angeordnet sein, um ein Herausdrehen der Schraube 5 aus dem Grundkörper 3 zu verhindern.

Weiterhin ist innerhalb des Grundkörpers 3 ein elastisches Element 4, im dargestellten Ausführungsbeispiel in Gestalt eines Tellerfederpaketes, angeordnet, auf das die Einstellschraube 5 einwirkt. Über Hineindrehen der Einstellschraube 5 in Richtung auf das elastische Element 4 wird dieses komprimiert. Die dadurch entstehende Federkraft wird über eine Aufnahmeeinrichtung 64 weiter radial nach außen geleitet. An der Außenseite der Aufnahmeeinrichtung 64 ist das Kontaktelement 6 oder sind die Kontaktelemente 6 angeordnet, über die dann die Druckkraft des elastischen Elementes 4 oder des Federpaketes auf die Zylinderinnenwand ausgeübt wird. Je nach Federvorspannung ändert sich der Anpressdruck des Kontaktelementes 6 auf die Zylinderinnenwand und damit der Reibungswiderstand, der bei einer Bewegung des Kolbens 10 innerhalb des Zylinders 2 erzeugt wird.

Figur 3 zeigt eine Detailansicht der Figur 2 ohne die Bohrung 35, die den Zugang zu der Einstellschraube 5 ermöglicht. Die Einstellschraube 5 ist in dem Gewindeeinsatz 51 eingeschraubt und wirkt über eine Druckplatte 41 auf das Tellerfederpaket als elastisches Element 4. Alternativ zu einer Tellerfederanordnung kann eine Schraubenfeder, eine Schraubentellerfeder oder auch ein Elastomerelement, beispielsweise aus TPU, verwendet werden. Ebenfalls ist es möglich eine Kombination verschiedener Federelemente oder Federtypen in Reihe anzuordnen, um unterschiedliche Federkennlinien zu erzeugen oder das gewünschte elastische Verhalten zu erreichen. Das elastische Element 4 wiederum übt Druck auf die Aufnahmeeinrichtung 64 aus, die längsverschieblich in Radialrichtung nach außen in dem Grundkörper 3 gelagert ist. In der Aufnahmeeinrichtung 4 ist eine Ausnehmung 644 ausgebildet, in der das elastische Element 4 eingesetzt ist. Die Ausnehmung 644 ist beispielsweise als Bohrung oder kreiszylindrische Ausnehmung ausgebildet, in die entsprechend runde Druckscheiben 41 und runde Tellerfederelemente eingesetzt werden können. Die Ausnehmung oder Bohrung 644 dient zur Führung der Federelemente. Alternativ zu einer Bohrung kann auch ein Zapfen oder ähnliches an der Aufnahmeeinrichtung 64 angeordnet oder ausgebildet sein, um das elastische Element 4 aufzunehmen.

Wird die Einstellschraube 5 in Richtung auf das elastische Element 4 eingeschraubt, erhöht sich die Federvorspannung und damit der Druck, mit der das Kontaktelement 6 und korrespondierend die Gegenfläche 6'an die Zylinderwand angepresst wird.

Über das Gewinde der Einstellschraube 5 lässt sich eine sehr einfache und genaue Einstellung der aufzubringenden Reibungskraft erreichen. Das elastische Element 4 bewirkt eine Kompensation eines Verschleißes des Kontaktelementes 6 bei länger dauernder Benutzung, so dass eine automatische Nachstellung der Vorspannkraft erreicht wird. Dadurch ist es möglich, eine dauerhafte, nahezu konstante Reibkraft über das Kontaktelement auf den Zylinder aufzubringen und damit einen definierten Widerstand gegen eine Kolbenbewegung innerhalb des Zylinders 2 zu gewährleisten.

Bei einem weitergehenden Verschleiß kann das Kontaktelement 6 ausgetauscht werden. Hierbei ist es möglich, durch Einsetzen weiterer Tellerfederpakete oder durch Austausch der elastischen Elemente 4 unterschiedliche Kraftniveaus einzustellen. Der Austausch kann sehr einfach erfolgen.

Da in dem Zylinder 2 keine Flüssigkeiten bewegt werden müssen, kann die Zylinderwand unterbrochen sein, wie in der Figur 4 dargestellt. Durch eine Zugangsöffnung 25 in der Zylinderwand ist es möglich, während des Betriebes oder im montierten Zustand des Zylinders 2 eine Veränderung oder Anpassung der Federvorspannung vorzunehmen. Ebenfalls wird durch eine solche Öffnung 25 die Montage erleichtert, da der Kolben 3 zunächst ohne Vorspannung des Kontaktelementes 6 in den Zylinder 2 eingeführt werden kann. So kann ohne eine Vorspannung zunächst die Aufnahmeeinrichtung 4 zusammen mit dem Kontaktelement 6 vollständig zur Anlage an dem Grundkörper 3 gebracht werden, so dass das Kontaktelement 6 bündig mit der Außenseite 31 des Kolbens 10 abschließt. Nach dem Einführen in den Zylinder 2 kann dann die Federvorspannung durch Einschrauben der Einstellschraube 3 in Richtung auf das elastische Element eingestellt werden.

Die Erfindung nutzt die Formänderung des elastischen Elementes 4, um eine stufenlos einstellbare Bremskraft zu erzeugen. Das elastische Element 4 selbst ist verschleißfrei, ein Verschleiß des Kontaktelementes 6 wird durch das elastische Element ausgeglichen. Durch Nachstellen der Einstellschraube 5 kann eine nachlassende Federkraft ausgeglichen werden.

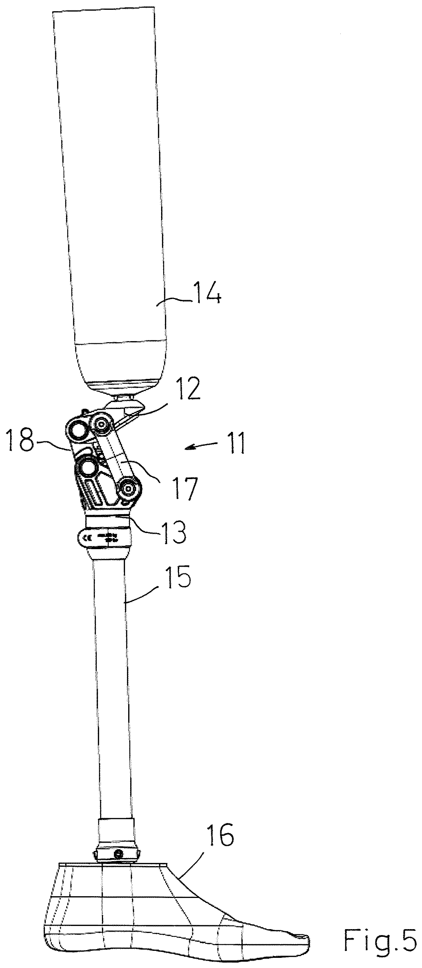

In der Figur 5 ist in einer Gesamtansicht ein Prothesenbein mit einem Prothesenkniegelenk 1 mit einem Oberteil 12 und einem gelenkig daran befestigten Unterteil 13 gezeigt. An dem Oberteil 12 ist eine Aufnahmeeinrichtung 14 in Gestalt eines Prothesenschaftes oder eines Liners zur Aufnahme eines Oberschenkelstumpfes befestigt, an dem Unterteil 13 ein Unterschenkelrohr 15, an dessen distalem Ende ein Prothesenfuß 16 angeordnet ist. Das Prothesengelenk 11 als Prothesenkniegelenk ist ein Mehrlenkergelenk mit einem vorderen Lenker 17 und einem hinteren Lenker 18, die über zwei Gelenkachsen mit dem Oberteil 12 und dem Unterteil 13 verbunden sind.

In der Figur 4, in der das Prothesenkniegelenk 11 in einer Schnittdarstellung gezeigt ist, ist das Oberteil 12 und das Unterteil 13 zu erkennen. Innerhalb des Unterteils 13 ist der Zylinder 2 angeordnet, in dem der Kolben 10 über die Kolbenstange 7 entgegen einer Federkraft, die über die Feder 19 auf den Kolbenboden ausgeübt wird, in distale Richtung, also in Richtung auf den Prothesenfuß, verlagert wird, wenn das Prothesenkniegelenk 11 eingebeugt wird. Bei einer Einbeugung oder Flexion verschwenken sowohl der vordere Lenker 17 als auch der hintere Lenker 18 um ihre jeweiligen Schwenkachsen und führen eine Drehung entgegen dem Uhrzeigersinn bei der Ansicht gemäß Figur 4 aus. Dadurch wird ein Zapfen, der sich an dem hinteren Lenker 18 befindet, ebenfalls entgegen dem Uhrzeigersinn verlagert, wodurch sich neben einer Verschwenkung der Kolbenstange 7 um den Kolbenbolzen 8 auch eine Kraftkomponente entgegen der Kraftrichtung der Feder 19 ergibt. Der Kolben 10 wird dann nach unten gedrückt. Wird das Prothesenkniegelenk 11 wieder gestreckt, drückt die Feder 19 den Kolben 10 nach oben in Richtung auf das Oberteil 12.

Um die Widerstandskraft des Kolbens 10 gegen eine Verlagerung innerhalb des Zylinders 2 einzustellen, kann durch die Öffnung 25 und die Bohrung 35 die Einstellschraube 5 in Richtung auf das Kontaktelement 6 verlagert werden, so dass über das Kontaktelement 6 und die Gegenfläche 6' an dem Kolben 10 eine vergrößerte Anpresskraft und damit eine vergrößerte Reibwirkung des Kolbens einstellbar ist.

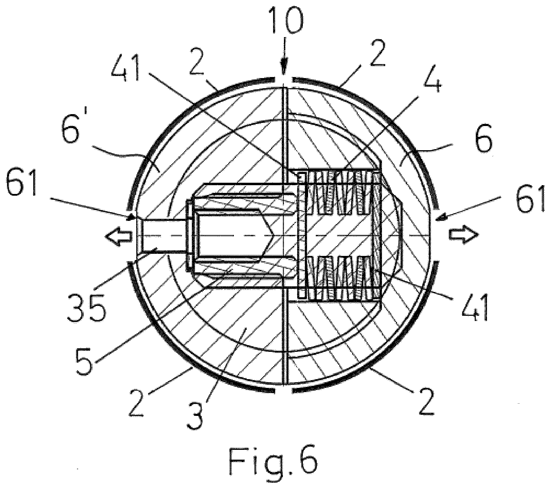

In Figur 6 ist eine Schnittdarstellung durch einen Kolben 10 gezeigt, bei der der Schnitt senkrecht zu der Kolbenhubrichtung gelegt ist. Der Zylinder 2 ist nur teilweise dargestellt, nämlich in Gestalt aktiver Reibflächen, die die Kontaktflächen zwischen dem Kolben 10 und dem Zylinder 2 ausbilden. An dem Kolben 10 ist das Kontaktelement 6 beweglich gelagert. Das elastische Element 4 in Gestalt einer Feder ist über zwei Druckplatten 41 elastisch in Richtung auf die aktiven Reibflächen des Zylinders 2 vorgespannt, die Wirkrichtung der Anpresskraft auf das Kontaktelement 6 ist durch einen Pfeil dargestellt. Der entgegengesetzt gerichtete Pfeil deutet die Wirkrichtung der Anpresskraft der Gegenfläche 6' gegen die zugeordneten aktiven Reibflächen des Zylinders 2 an. Innerhalb des Grundkörpers 3 ist die Einstellvorrichtung 5 in Gestalt einer Einstellschraube angeordnet. Die Einstellschraube 5 ist durch die Bohrung 35 von radial außen zugänglich, sodass sich die Vorspannung des elastischen Elementes 4 hinsichtlich des Kontaktelementes 6 verändern lässt. In dem dargestellten Ausführungsbeispiel wirkt die Andruckplatte 41, die an dem der Einstellschraube 5 abgewandten Ende des elastischen Elementes 4 angeordnet ist, unmittelbar auf das Kontaktelement 6 und drückt dieses gegen die aktiven Reibflächen des Zylinders 2. Die sich einstellende Reibungskraft wird zwischen dem Kontaktelement 6, der Gegenfläche 6' sowie den aktiven Reibflächen des Zylinders 2 erzeugt. Die höchsten Reibkräfte werden dabei in dem Bereich der Wirkrichtung der Vorspannung durch das elastische Element 4 erzielt und nehmen in Umfangsrichtung zur Seite hin ab. Idealerweise weisen die Radien des Kolbens 10 mit der Gegenfläche 6' und dem Kontaktelement 6 sowie dem Zylinder 2 die gleichen Abmessungen auf, wobei eventuell auftretende Toleranzen durch elastische Verformung der Komponenten kompensiert werden können.

In dem dargestellten Ausführungsbeispiel der Figur 6 ist in dem Bereich der Wirkrichtung auf einander gegenüberliegenden Seiten jeweils eine Abflachung 61 angeordnet, sodass sich in dem Bereich der Bohrung 35 sowie auf der der Bohrung 35 gegenüberliegende Seite des Kolbens 10 jeweils eine Abflachung 61 in dem Grundkörper 3 in der Gegenfläche 6' und dem Kontaktelement 6 ergibt. Die teilweise über den Umfang ausgebildeten Abflachungen 61 in diesen Bereichen an dem Kontaktelement 6 und der Gegenfläche 6' vermeiden eine hohe Flächenpressung in diesen Bereichen und verteilen die wirksamen Anpresskräfte besser und gleichmäßiger über den Umfang des Kolbens 10 und des Kontaktelementes 6. Ohne die Abflachungen 61 würde eine erhöhte Anpressung vordringlich auf der Kraftwirkungslinie des elastischen Elementes 4 wirken, wenn der Radius des Kolbens 10 geringfügig kleiner als der Radius des Zylinders 2 ist.

## Patentansprüche

1. Orthopädietechnische Einrichtung mit einem Kolben (10) zur Anordnung in einem Dämpfer (1) und einem Zylinder (2), in dem der Kolben (10) relativ zu dem Zylinder (2) verlagerbar gelagert ist, wobei der Kolben (10) einen Grundkörper (3) aufweist, an dessen Außenseite (31) zumindest ein Kontaktelement (6) angeordnet ist, das in Richtung auf den Zylinder (2) verlagerbar und mit einem elastischen Element (4) gekoppelt ist, wobei das elastische Element (4) über eine Einstellschraube (5) entlang der Verlagerungsrichtung des Kontaktelementes (6) entlang einer Kraftwirkungslinie vorspannbar ist, **dadurch gekennzeichnet, dass** in dem Grundkörper (3) eine Bohrung (35) eingearbeitet oder ausgebildet ist, die sich radial nach innen erstreckt, so dass die Einstellschraube (5) in radialer Richtung entlang der Kraftwirkungslinie auf das elastische Element (4) hin verlagert werden kann, wobei die dadurch entstehende Federkraft weiter radial nach außen zu dem Kontaktelement (6) geleitet wird.

2. Orthopädietechnische Einrichtung nach Anspruch 1, **dadurch gekennzeichnet, dass** das elastische Element (4) eine Tellerfederanordnung, Schraubentellerfeder, Schraubenfeder und/oder ein Elastomer umfasst.

3. Orthopädietechnische Einrichtung nach einem der voranstehenden Ansprüche, **dadurch gekennzeichnet, dass** eine Aufnahmeeinrichtung (64) für das elastische Element (4) an dem Kontaktelement (6) angeordnet, befestigt oder ausgebildet ist.

4. Orthopädietechnische Einrichtung nach Anspruch 3, **dadurch gekennzeichnet, dass** die Aufnahmeeinrichtung (64) verlagerbar in dem Grundkörper (3) angeordnet ist.

5. Orthopädietechnische Einrichtung nach Anspruch 3 oder 4, **dadurch gekennzeichnet, dass** die Aufnahmeeinrichtung (64) eine Ausnehmung (644) aufweist, in der das elastische Element (4) eingeführt ist.

6. Orthopädietechnische Einrichtung nach einem der voranstehenden Ansprüche, **dadurch gekennzeichnet, dass** das Kontaktelement (6) sich über mehr als die Hälfte der Höhe des Grundkörpers (3) erstreckt.

7. Orthopädietechnische Einrichtung nach einem der voranstehenden Ansprüche, **dadurch gekennzeichnet, dass** die Einstellschraube (5) in dem Grundkörper (3) gelagert ist.

8. Orthopädietechnische Einrichtung nach einem der voranstehenden Ansprüche, **dadurch gekennzeichnet, dass** eine Reibkraft für die Dämpfung einer Bewegung zwischen dem Kolben (10) und dem Zylinder (2) erzeugt wird.

9. Orthopädietechnische Einrichtung nach Anspruch 8, **dadurch gekennzeichnet, dass** die Reibkraft über die Oberfläche des Kontaktelementes (6), dessen Gegenfläche (6') an dem Grundkörper (3) und der Zylinderinnenwand erzeugt wird.

10. Orthopädietechnische Einrichtung nach einem der voranstehenden Ansprüche, **dadurch gekennzeichnet, dass** an dem Kolben (3) und/oder dem Kontaktelement (6) zumindest eine Abflachung (61) ausgebildet ist.

11. Orthopädietechnische Einrichtung nach Anspruch 10, **dadurch gekennzeichnet, dass** zumindest eine aktive Reibfläche zwischen dem Kolben (3), dem Kontaktelement (6) und dem Zylinder (2) in Umfangsrichtung neben der Abflachung (61) ausgebildet ist.

12. Dämpfer (1) für eine orthopädietechnische Einrichtung, mit einem Zylinder (2) und einem beweglich darin gelagerten Kolben (10) nach einem der voranstehenden Ansprüche.

## Claims

1. Orthopedic device with a piston (10) for arrangement in a damper (1) and a cylinder (2), in which the piston (10) is mounted displaceably relative to the cylinder (2), wherein the piston (10) has a base body (3) on the outer side (31) of which at least one contact element (6) is arranged, which is displaceable in a direction toward the cylinder (2) and is coupled to an elastic element (4), wherein the elastic element (4) can be preloaded via an adjusting screw (5) along the direction of displacement of the contact element (6) along force action line, **characterized in that** a bore (35) is formed or provided in the base body (3), the bore (35) extending radially inward, such that the adjusting screw (5) can be displaced in a radial direction along the force action line toward the elastic element (4), the resulting spring force being transmitted further radially outwards to the contact element (6).

2. Orthopedic device according to claim 1, **characterized in that** the elastic element (4) comprises a disc spring arrangement, a screw disc spring, a coil spring, and/or an elastomer.

3. Orthopedic device according to one of the preceding claims, **characterized in that** a receiving device (64) for the elastic element (4) is arranged, fastened, or formed on the contact element (6).

4. Orthopedic device according to claim 3, **characterized in that** the receiving device (64) is arranged displaceably in the base body (3).

5. Orthopedic device according to claim 3 or 4, **characterized in that** the receiving device (64) has a recess (644) into which the elastic element (4) is inserted.

6. Orthopedic device according to one of the preceding claims, **characterized in that** the contact element (6) extends over more than half the heigh of the base body (3).

7. Orthopedic device according to one of the preceding claims, **characterized in that** the adjusting screw (5) is mounted in the base body.

8. Orthopedic device according to one of the preceding claims, **characterized in that** a frictional force is generated for damping a movement between the piston (10) and the cylinder (2).

9. Orthopedic device according to claim 8, **characterized in that** the frictional force is generated via the surface of the contact element (6), its counter surface (6') on the base body (3), and the inner wall of the cylinder.

10. Orthopedic device according to one of the preceding claims, **characterized in that** at least one flattened portion (61) is formed on the piston (3) and/or the contact element (6).

11. Orthopedic device according to claim 10, **characterized in that** at least one active friction surface is formed between the piston (3), the contact element (6), and the cylinder (2) in the circumferential direction next to the flattened portion (61).

12. Damper (1) for an orthopedic device, with a cylinder (2) and a piston (10) moveably mounted therein, according to one of the preceding claims.

## Revendications

1. Dispositif orthopédique comprenant un piston (10), destiné à être disposé dans un amortisseur (1), et un cylindre (2) dans lequel le piston (10) est monté de manière déplaçable par rapport au cylindre (2), le piston (10) présentant un corps de base (3) sur la face extérieure (31) duquel est disposé au moins un élément de contact (6) qui est déplaçable en direction du cylindre (2) et est couplé à un élément élastique (4), l'élément élastique (4) pouvant être précontraint le long d'une ligne d'action des forces selon la direction de déplacement de l'élément de contact (6) par l'intermédiaire d'une vis de réglage (5),
**caractérisé en ce qu'**un alésage (35) s'étendant radialement vers l'intérieur est ménagé ou formé dans le corps de base (3), de sorte que la vis de réglage (5) peut être déplacée vers l'élément élastique (4) dans la direction radiale le long de la ligne d'action des forces, la force élastique ainsi générée étant dirigée vers l'élément de contact (6) radialement vers l'extérieur.

2. Dispositif orthopédique selon la revendication 1,
**caractérisé en ce que** l'élément élastique (4) comprend un ensemble à rondelle ressort, une rondelle ressort hélicoïdale, un ressort hélicoïdal et/ou un élastomère.

3. Dispositif orthopédique selon l'une des revendications précédentes,
**caractérisé en ce qu'**un dispositif de réception (64) prévu pour l'élément élastique (4) est disposé, fixé ou formé sur l'élément de contact (6).

4. Dispositif orthopédique selon la revendication 3,
**caractérisé en ce que** le dispositif de réception (64) est disposé de manière déplaçable dans le corps de base (3).

5. Dispositif orthopédique selon la revendication 3 ou 4,
**caractérisé en ce que** le dispositif de réception (64) présente un évidement (644) dans lequel est inséré l'élément élastique (4).

6. Dispositif orthopédique selon l'une des revendications précédentes,
**caractérisé en ce que** l'élément de contact (6) s'étend sur plus de la moitié de la hauteur du corps de base (3).

7. Dispositif orthopédique selon l'une des revendications précédentes,
**caractérisé en ce que** la vis de réglage (5) est logée dans le corps de base (3).

8. Dispositif orthopédique selon l'une des revendications précédentes,
**caractérisé en ce qu'**une force de frottement pour amortir un mouvement est générée entre le piston (10) et le cylindre (2).

9. Dispositif orthopédique selon la revendication 8,
**caractérisé en ce que** la force de frottement est générée par l'intermédiaire de la surface de l'élément de contact (6), de la surface antagoniste (6') située sur le corps de base (3), et de la paroi intérieure du cylindre.

10. Dispositif orthopédique selon l'une des revendications précédentes,
**caractérisé en ce qu'**au moins un méplat (61) est formé sur le piston (10) et/ou sur l'élément de contact (6).

11. Dispositif orthopédique selon la revendication 10,
**caractérisé en ce qu'**au moins une surface de frottement active entre le piston (10), l'élément de contact (6) et le cylindre (2) est formée à côté du méplat (61) dans la direction circonférentielle.

12. Amortisseur (1) pour un dispositif orthopédique selon l'une des revendications précédentes, comprenant un cylindre (2) et un piston (10) monté de manière mobile dans celui-ci.
